# EUROPEAN PATENT APPLICATION

(11) **EP 4 670 612 A1**
(43) Date of publication of application: **31.12.2025**
(21) Application number: 25182433.0
(22) Date of filing: 12.06.2025
(51) Int. Cl.: A61B 1/00, A61B 1/12, A61B 1/24

(54) **AN INTRAORAL SCANNING DEVICE WITH IMPROVED COOLING**

(30) Priority: 26.06.2024 DK PA202470178; 02.10.2024 DK PA202470248
(71) Applicant: 3Shape A/S, 1060 Copenhagen K (DK)
(72) Inventor: HANSEN, Finn, 1060 Copenhagen K (DK)
(74) Representative: Guardian IP Consulting I/S

(57) **Abstract**

This disclosure relates to an intraoral scanning device (100, 200, 300, 400, 600) configured to perform a scanning of a dental object inside an oral cavity, the device comprising: one or more electronic units (104); a cooling system (106) configured to remove heat from the one or more electronic units, the cooling system comprising a heat exchanger unit (108) and a flow generating unit (110), wherein at least a part of the flow generating unit is covered by the heat exchanger unit; an air inlet (112) and an air outlet (114), wherein the air inlet and air outlet are separated by the heat exchanger unit, wherein the flow generating unit is configured to move air between the air inlet and the air outlet and through the heat exchanger unit in a direction mainly perpendicular to a longitudinal axis of the intraoral scanning device.

## Description

### TECHNICAL FIELD

The present disclosure relates to an intraoral scanning device with an integrated cooling system, more specifically, the disclosure relates to an improved cooling system that provides an improved cooling within the intraoral scanning device.

### BACKGROUND OF THE INVENTION

Nowadays, intraoral scanning technology is widely used to acquire information on a patient's oral cavity. Typically, an intraoral scanning device includes a plurality of electronic components that are tightly packed within a housing of the device to maximize the power efficiency and minimize energy losses. Hence, thermal energy tends to locally concentrate in specific regions along the intraoral scanning device, causing the heating of the exterior surface of the device in said regions. Safety regulations impose that the temperature of the exterior surface of a medical device must not exceed predefined limits, and in particular the exterior surface of handheld intraoral scanning devices must not exceed the temperature of 43 degrees Celsius. The thermal management of intraoral scanning devices is therefore crucial to guarantee the safety of a user performing a scanning of the patient's oral cavity. As technology advances, the plurality of electronic components comprised within intraoral scanning devices is becoming more powerful and accurate in the acquisition and the processing of data relative to the patient's oral cavity, e.g. due to higher frame rate image sensors, wireless operations, processing electronics, and so forth. Such an increase in power naturally results in an increase of the thermal energy localized along the housing of the device, and said thermal energy must be efficiently dissipated to guarantee the safety of the user of the intraoral scanning device. At the same time, the size of intraoral scanning devices is decreasing, with the aim to provide both a more comfortable scanning experience for the patient and a more ergonomic intraoral scanning device for the user performing the scanning. Therefore, it appears clear that one of the major challenges is to guarantee that both thermal management and electronic power are optimized within increasingly smaller intraoral scanning devices.

Current intraoral scanning devices already include an integrated cooling system, wherein said cooling system is arranged in the housing of the device in such a way that thermal contact between the cooling system and the electronic components of the device is achieved. To ensure maximal hygiene for the patient being scanned, a battery can be inserted and removed from a rear end of the intraoral scanning device, for example to recharge the battery and/or to replace the battery with a new battery. Thus, the cooling system is permanent mounted within the housing of the intraoral scanning device and arranged in such a way that, when the battery is inserted from the rear end of the device, the cooling system is arranged in vicinity to a front end of the battery that is arranged furthest inside the device than an opposite end of the battery. With such an arrangement of components, thermal management of the intraoral scanning device is achieved by sucking air through an air inlet of the intraoral scanning device, letting air pass through the cooling system and blowing air out from an air outlet of the device. Said air inlet and air outlet are typically separated, at least partly, by the battery, and located in a rear end of the intraoral scanning device, such that air moves along a first passage which extends from the air inlet to the cooling system, and, after passing through the cooling system, air moves along a second air passage which extends from the cooling system to the air outlet.

A first disadvantage of current solutions to cool the housing of intraoral scanning devices is that inlet air moves in the first air passage along a first direction, and, after passing through the cooling system, outlet air moves in the second air passage along a second direction which is substantially parallel and opposite to the first direction. This implies that the air flow must turn an angle of 90 degrees when going from the air inlet to the cooling system and the air flow must turn an additional angle of 90 degrees when going from the cooling system to the air outlet. Thus, it is inevitable that at least a part of the air flow hits and bounces off a surface of the housing of the intraoral scanning device after passing through the cooling system, when going from the cooling system to the air outlet. The collision of outlet air bounced in different directions causes the development of a pressure drop across the cooling system, which in turn generates air turbulences in the outlet air, thereby significantly decreasing the efficiency of the cooling process.

A second disadvantage of the cooling system integrated within current intraoral scanning devices is that the air inlet and the air outlet are separated by the battery, i.e. the first air passage typically extends along a side of the battery of the intraoral scanner device. Since the battery heats up while the intraoral scanning device is in use, the air sucked in the cooling system through the air inlet is pre-heated by the battery, further reducing the efficiency of the cooling process.

All of these suggest the need for an improved intraoral scanning device to overcome the above-mentioned disadvantages of current intraoral scanning devices and thus optimize the efficiency of the cooling system of said devices.

### SUMMARY

It is one aspect of the present disclosure to provide an intraoral scanning device that overcomes the above-mentioned disadvantages. The intraoral scanning device comprises one or more electronic units and a cooling system. The cooling system may be configured to remove heat from the one or more electronic units, and furthermore, the cooling system includes a heat exchanger unit and a flow generating unit, wherein at least a part of the flow generating unit is covered by the heat exchanger unit. Said intraoral scanning device further comprises an air inlet and air outlet which may be separated by the heat exchanger unit, such that the flow generating unit may be configured to move air between the air inlet and the air outlet and through the heat exchanger unit in a direction mainly perpendicular to a longitudinal axis of the intraoral scanning device. Such an arrangement of the cooling system relative to the air inlet and the air outlet ensures that, after passing through the cooling system, the outlet air flow does not encounter any surface of a housing of the intraoral scanning device, since the air flow does not need to turn an angle of 90 degrees when air flows from the air inlet through the heat exchanger unit in a direction towards the air outlet, thereby preventing the development of a pressure drop across the cooling system and turbulences along the outlet air flow. Additionally, air flows from the air inlet through the heat exchanger unit without encountering any heated component of the device, such as a battery, i.e. the inlet air flow is not pre-heated before passing through the cooling system. In other words, air may enter the heat exchanger directly through the air inlet and leave the heat exchanger directly through the air outlet, moving along the same direction mainly perpendicular to the longitudinal axis, i.e. air does not change direction of motion while moving between the air inlet and the air outlet through the heat exchanger. Therefore, contrary to currently existing intraoral scanning devices, in the intraoral scanning device of the present disclosure air encounters neither a first passage extending from the air inlet to the heat exchanger nor a second passage extending from the heat exchanger to the air outlet, wherein the first passage and the second passage may be parallel to the longitudinal axis of the intraoral scanning device. Thus, the cooling efficiency is optimized, and the safety of the intraoral scanning device is substantially enhanced for a user performing the scanning.

Accordingly, in one aspect there is provided herein an intraoral scanning device configured to perform a scanning of a dental object inside an oral cavity. The intraoral scanning device may comprise one or more electronic units. The intraoral scanning device may further comprise a cooling system configured to remove heat from the one or more electronic units. The intraoral scanning device may further comprise an air inlet and an air outlet. The air inlet and the air outlet may be separated by the cooling system. The cooling system may be configured to move air between the air inlet and the air outlet and through the cooling system in a direction mainly perpendicular to a longitudinal axis of the scanner. In other words, the cooling system may be configured to move air between the air inlet and the air outlet and through the cooling system in such a way that air does not change direction during its motion, i.e. an angle between the direction of air and the longitudinal axis of the intraoral scanning device is mainly 90° throughout the passage of air between the air inlet and the air outlet through the heat exchanger. It is therefore an advantage that the air inlet and the air outlet are separated by the cooling system, as the air flow does not encounter any surface of a housing of the intraoral scanning device when moving from the air inlet to the air outlet and through the cooling system, thereby preventing the development of turbulences along the outlet air flow and optimizing the cooling efficiency of the device.

The intraoral scanning device may be configured to perform a scanning of a dental object inside an oral cavity. The dental object may be a patient's dentition, gingiva, but also dental fillings, restorations, preparation sites, implants, or any other object related to dentistry. The intraoral scanning device may employ a scanning principle such as triangulation-based scanning, confocal scanning, focus scanning, ultrasound scanning, x-ray scanning, stereo vision, structure from motion, optical coherent tomography (OCT), or any other scanning principle. The intraoral scanning device may be configured to acquire data relative to the patient's oral cavity, wherein said data comprises information relating to the 3D dental object. The data acquired by the intraoral scanning device may comprise: two-dimensional (2D) images, 3D point clouds, depth data, texture data, intensity data, color data, and/or combinations thereof. The intraoral scanning device may be configured to process, partially or completely, the acquired data to generate a three-dimensional (3D) digital model of at least a part of the patient's oral cavity based on said acquired data.

The intraoral scanning device may comprise one or more electronic units. The one or more electronic units may comprise a projector unit that is configured to project light onto the dental object in the oral cavity of the patient. For example, the projector unit may be a Digital Light Processing (DLP) projector that uses a micro mirror array for generating a time varying pattern, or the projector unit may be a diffractive optical element (DOF), or a back-lit mask projector comprising a light source that is placed behind a mask that has a special pattern, such that the light projected onto the dental object is patterned. The projector unit may comprise at least one light source that is configured to generate light of a single wavelength and/or of a combination of wavelengths (mono- or polychromatic). For example, the at least one light source may be at least one Light Emitting Diode (LED) unit. The one or more electronic units may further comprise an image sensor that is configured to capture the light reflected from the scanned dental object, and to generate a plurality of images based on the captured light. For example, the image sensor may be a monochrome sensor which comprises a color filter array, such as a Bayer filter and/or additional filters. The one or more electronic units may comprise one or more processors that are configured to process, partially or completely, the data acquired by the intraoral scanning device during the scanning of the dental object in the oral cavity. For example, the one or more processors may comprise a Field-programmable gate array (FPGA) and/or an Advanced RISC Machine (ARM). Typically, the at least one light source, the image sensor and the one or more processors constitute the main sources of heat for the intraoral scanning device. It is therefore important to dissipate the heat generated by said sources while the intraoral scanning device is in use.

The intraoral scanning device may comprise a cooling system configured to remove heat from the one or more electronic units, wherein the cooling system may comprise a heat exchanger unit and a flow generating unit, and wherein at least a part of the flow generating unit may be covered by the heat exchanger unit. The heat exchanger unit may be a heatsink that comprises fins arranged on at least two opposite sides of the heatsink, wherein between the two opposite sides a cavity is arranged such that the flow generating unit may slide through said cavity until at least a part of the flow generating unit is covered by the heatsink. The flow generating unit may be a fane unit, such as an axial-flow fan that has blades defining an axis of rotation. The flow generating unit may further or alternatively be a blower, or a solid stage cooler, or a piezo cooler, and so forth. Thus, the flow generating unit may be configured to force air to move in a direction which is mainly perpendicular to a longitudinal axis of the intraoral scanning device, wherein the longitudinal axis of the intraoral scanning device extends between a tip end of the intraoral scanning device, i.e. the end of the intraoral scanning device for entering the patient's oral cavity, and a rear end of the intraoral scanning device, wherein the rear end is opposite to the tip end. In other words, air is forced to move along a direction which forms an angle of 90° with the longitudinal axis of the intraoral scanning device at the entrance of the air inlet, during the passage through the heat exchanger, and at the exit from the air outlet. The direction in which the air is forced to move by the flow generating unit may further be parallel with a horizontal plane of the intraoral scanning device. Advantageously, the heat exchanger unit enables the intraoral scanning device to dissipate heat from the one or more electronic units. Furthermore, the flow generating unit is configured to generate a flow of air through the heat exchanger unit, thereby cooling the heat exchanger unit.

The intraoral scanning device may comprise an air inlet and an air outlet, wherein the air inlet and the air outlet are separated by the heat exchanger unit. Advantageously, this ensures that inlet air directly flows through the heat exchanger unit without first encountering any component of the device which is heated while the device is in use, e.g. a battery of the device. Thus, the inlet air flow is not pre-heated before passing through the heat exchanger unit, thereby enhancing the cooling efficiency of the cooling system of the intraoral scanning device.

The flow generating unit may be configured to move air between the air inlet and the air outlet and through the heat exchanger unit in a direction mainly perpendicular to a longitudinal axis of the intraoral scanning device. Thus, air moves from the air inlet to the heat exchanger unit along a first direction perpendicular to a longitudinal axis of the scanner, and, after passing through the heat exchanger unit and exchanging heat with the heat exchanger unit, air moves from the heat exchanger unit to the air outlet along a second direction perpendicular to a longitudinal axis of the scanner. Advantageously, the second direction may be the same as the first direction, such that the air flow does not need to turn when moving from the air inlet to the air outlet. In other words, air enters the heat exchanger through the air inlet by moving along a direction perpendicular to the longitudinal axis of the intraoral scanning device, air moves through the heat exchanger along the same direction perpendicular to the longitudinal axis of the intraoral scanning device, and air exits the heat exchanger through the air outlet along the same direction perpendicular to the longitudinal axis of the intraoral scanning device, i.e. air does not change direction while moving between the air inlet and air outlet and through the heat exchanger. This ensures that the air flow does not encounter any surface of the housing of the intraoral scanning device when going from the heat exchanger unit to the air outlet, thereby preventing the development of a pressure drop across the heat exchanger unit and preventing the development of air turbulences along the outlet air flow. This may significantly enhance the cooling efficiency in the intraoral scanning device.

The one or more electronic units may comprise a projector unit that may be configured to project light onto the dental object in the oral cavity. The one or more electronic units may further comprise an image sensor that is configured to capture reflected light from the dental object in the oral cavity. The one or more electronic units may further comprise one or more processors that are configured to process a plurality of data acquired by the scanning device during the scanning of the dental object in the oral cavity. The projector unit, the image sensor and the one or more processors are the main sources of heat within the intraoral scanning device, hence removing from the intraoral scanning device excess heat produced by said main sources of heat is important.

The intraoral scanning device may comprise a battery unit that is configured to power the flow generating unit via at least one electrical connection or at least one inductive connection. The at least one electrical connection may be realized by means of one or more conductive wires, i.e. the flow generating unit may be attached to a power unit of the battery by means of one or more conductive wires. The at least one electrical connection may further or alternatively be realized by means of a Universal Serial Bus (USB) connection, i.e. the flow generating unit may be connected to a power unit of the battery by means of a USB plug, wherein the USB plug may be comprised in the flow generating unit and a USB port may be comprised in the battery unit. Advantageously, the at least one electrical connection can easily be implemented in existing intraoral scanning device configurations. The at least one inductive connection may be realized by means of an electromagnetic field that may be generated within the power unit of the battery, e.g. through an alternating current that flows through an inductor (such as a transmitter coil) comprised within the power unit of the battery. The electromagnetic field may then be received through a receiver unit comprised within the flow generating unit, e.g. through a receiver coil. Advantageously, the at least one inductive connection is wireless, whereby one or more wires connecting the flow generating unit to the battery unit may be damaged and/or break, and may therefore need maintenance. Thus, the inductive connection may reduce the costs of maintenance of the intraoral scanning device. Furthermore, the at least inductive connection provides an easy dissembling of the flow generating unit from the battery unit since no wires need to be disconnected before dissembling the flow generating unit. In this example, the flow generating unit may be connected to the battery unit by one or more magnetic means that may include a ferromagnetic or ferrimagnetic material.

The intraoral scanning device may comprise a battery unit, and the flow generating unit may be permanent mounted to the battery unit or the flow generating unit may be detachable mounted to the battery unit via magnetic force or a snap couple. When the flow generating unit is permanently mounted to the battery unit, i.e. the flow generating unit is integrated into the battery, the flow generating unit and the battery unit can be effectively regarded as a single component of the intraoral scanning device. Advantageously, it is practical and easy for the user to insert and/or remove said single component from the rear end of the intraoral scanner. When the flow generating unit is detachable mounted to the battery unit via magnetic force or a snap couple, it is an advantage that a single flow generating unit may be used for multiple battery units configured to power said flow generating unit, thereby reducing the cost of production of the intraoral scanner.

The intraoral scanning device may comprise a battery unit, and the heat exchanger unit may be arranged at the rear end of the intraoral scanning device allowing the battery unit to slide through the heat exchanger unit until at least one part of the flow generating unit is covered by the heat exchanger unit. This enables the cooling system to be arranged in vicinity to the rear end of the intraoral scanning device, while still ensuring that the battery unit can be inserted into the intraoral scanning device from the rear end of the intraoral scanning device and slide through the heat exchanger unit. In particular, this also enables the flow generating unit to be mounted to a rear end of the battery unit, such that the battery unit can slide through the heat exchanger unit until at least a part of the rear end of the battery unit, i.e. the part comprising the flow generating unit, is covered by the heat exchanger unit. Advantageously, the arrangement of the battery unit relative to the heat exchanger unit ensures that, when the intraoral scanning device is in use, the inlet air does not encounter the heated battery unit before passing through the cooling system, thereby enhancing the cooling efficiency. Another advantage of the battery unit being configured to slide through the heat exchanger unit is that the heat exchanger unit and/or the battery unit and/or the flow generating unit may comprise guiding rails which generate friction when the battery unit slides through the heat exchanger unit, thereby enabling the user to understand when the battery unit is correctly placed in the intraoral scanning device.

The flow generating unit may be configured to slide through the heat exchanger unit until at least a part of the flow generating unit is covered by the heat exchanger unit. This ensures that, when the flow generating unit is detachable mounted to the battery unit, the flow generating unit can be pulled out of the heat exchanger unit if it needs maintenance and/or needs to be replaced, without the need for replacing also the battery and/or the heat exchanger unit. Advantageously, this reduces the cost of maintenance of the intraoral scanning device.

The intraoral scanning device may comprise a battery unit, and the battery unit and the flow generating unit may be permanent or detachable mounted to a first housing of the device, and the battery unit and the flow generating unit may be maintained in the first housing when detaching the first housing from a second housing of the device, and wherein the second housing includes the one or more electronic components of the device. The user of the scanner may want and/or need to pull out the battery unit and/or the flow generating unit from the device for a number of reasons. For example, the user may need to recharge wirelessly the battery unit and/or to replace said battery unit with another battery unit. Alternatively, the user may want to replace and/or to clean the flow generating unit. It is therefore an advantage that the battery unit and the flow generating unit are mounted to the first housing of the intraoral scanning device, as this ensures that the battery unit and the flow generating unit can be pulled out from the intraoral scanning device while maintaining maximal hygiene for the patient being scanned. Additionally, the first housing ensures that the battery unit and the flow generating unit are protected from mechanical damage, whereas the second housing prevents the one or more electronic components from being damaged when pulling in and out the battery unit and/or the flow generating unit.

The battery unit or the flow generating unit may include an extractor, wherein the battery unit and the flow generating unit may be arranged within the first housing of the intraoral scanning device, and the extractor may be configured to pull out the battery unit and the flow generating unit upon a user pulling the extractor. If the battery unit and the flow generating unit are detachable mounted to the first housing of the intraoral scanning device, the extractor enables the user to extract the battery unit and/or the flow generating unit from the intraoral scanning device in a practical and easy way.

The intraoral scanning device may comprise a battery unit, and the battery unit may comprise a Universal Serial Bus (USB) port. The USB port may be arranged in an end-facet of the battery, wherein the end-facet may be arranged in vicinity to a rear end of the battery unit. For example, the USB port may be of type C, i.e. a USB-C port whose size may be suitable for the typical size of intraoral scanning devices. Advantageously, when the battery unit is extracted from the intraoral scanning device, the battery unit may be recharged upon connecting it to any charging unit comprising a USB plug compatible with the USB port comprised in the battery unit, i.e. a USB plug of the same type as the USB port, wherein the USB plug is configured to be connected to the USB port. Therefore, including the USB port in the battery unit ensures that the battery unit may be recharged by using any of a phone charger, a tablet charger, a headphone charger and so forth, configured to provide power through a USB plug compatible with the USB port of the battery unit, i.e. a USB plug configured to be connected to the USB port, whereby it is not required to produce and/or buy a specific charging unit for the intraoral scanning device, thereby reducing the cost of production and maintenance of the intraoral scanning device. This may have the further advantage of reducing a size of a box in which the intraoral scanning device is stored and/or sold, wherein said box is typically made of plastic or paper. In other words, including the USB port in the battery unit may reduce an environmental impact of the production of the intraoral scanning device, as the storage space required by the specific charger of the intraoral scanning device may not be needed, thereby reducing the amount of plastic and/or paper needed for producing the storage box of the intraoral scanning device.

The flow generating unit may comprise a USB plug configured to be connected to the USB port comprised in the battery unit, and wherein at least one electrical connection between the flow generating unit and the battery unit is obtained by the connection between the USB plug and the USB port. It is an advantage that the USB plug of the flow generating unit can be plugged into the USB port of the battery unit, as this ensures that once the battery unit is inserted into the intraoral scanning device the USB port is not exposed to the part of the intraoral scanning device which is in contact with a hand of the user of the intraoral scanning device, thereby ensuring that safety standards of medical devices are fulfilled.

The USB port may be arranged in a front-facet of a front end of the battery unit, wherein the front end may be arranged opposite to the rear end of the battery unit. The front-facet of the battery unit may be intended as the facet of the battery unit which faces the one or more electronic units when the battery unit is inserted into the intraoral scanning device. Advantageously, this ensures that when the battery unit is inserted into the intraoral scanning device, the USB port is not exposed to a hand of the user of the intraoral scanning device, thereby ensuring that safety standards of medical devices are fulfilled. Another advantage of including the USB port in the battery unit is that the battery unit may be recharged by using any of a phone charger, a tablet charger, a headphone charger and so forth, configured to provide power through a USB plug compatible with the USB port of the battery unit, i.e. a USB plug that is configured to be connected to the USB port, whereby it is not required to produce and/or buy a specific charging unit for the intraoral scanning device, thereby reducing the cost of production and maintenance of the intraoral scanning device. This may have the further advantage of reducing a size of a box in which the intraoral scanning device is stored and/or sold, wherein said box is typically made of plastic or paper. In other words, including the USB port in the battery unit may reduce an environmental impact of the production of the intraoral scanning device, as the storage space required by the specific charger of the intraoral scanning device may not be needed, thereby reducing the amount of plastic and/or paper needed for producing the storage box of the intraoral scanning device.

At least one of the one or more electronic units may comprise a USB plug configured to be connected to the USB port comprised in the battery unit, wherein at least one electrical connection between the flow generating unit and the battery unit is obtained by the connection between the USB plug and the USB port. Advantageously, the USB connection between the battery and the at least one or more electronic units may guide the user to place correctly the battery unit into the intraoral scanning device, i.e. to push the battery unit through the heat exchanger unit in the intraoral scanning device until the USB plug is correctly plugged into the USB port.

The heat exchanger unit may comprise two sides, and the heat exchanger unit may be arranged such that a first side faces a tip end of the intraoral scanning device, and a second side faces a rear end of the intraoral scanning device. The heat exchanger unit may be coupled to the one or more electronic units through at least one heat transferring unit, and the at least one heat transferring unit may be detachable or permanent mounted to the first side of the heat exchanger unit. Thus, the heat exchanger unit does not need to be placed in vicinity to the one or more electronic units to dissipate the heat produced by said electronic units, as the heat transferring unit is configured to transfer heat from the one or more electronic units to the heat exchanger unit. This enables the heat exchanger unit to be arranged in vicinity to the rear end of the intraoral scanning device, and it enables the air inlet to be arranged in vicinity to a first lateral facet of the heat exchanger unit and the air outlet to be arranged in vicinity to a second lateral facet of the heat exchanger unit, wherein the second lateral facet is opposite to the first lateral facet. Advantageously, with such an arrangement of components the air flow does not need to change direction when going from the air inlet to the air outlet, thereby preventing the development of air turbulence and pressure drop across the heat exchanger unit.

The second side of the heat exchanger unit may be mounted to a first housing of the intraoral scanning device that is detachable mounted to a second housing of the intraoral scanning device. The second housing may comprise the one or more electronic units of the intraoral scanning device. Advantageously, the heat exchanger unit can be pulled out from the device by means of extracting the first housing, such that the heat exchanger unit can be fixed and/or replaced in the case of a damage. This may reduce the costs of maintenance of the scanning device, since if the heat exchanger unit is damaged and/or broken there is no need to repair and/or replace the whole intraoral scanner device, but it is possible to repair and/or replace the heat exchanger unit only.

The at least one heat transferring unit may include one or more heat pipes, and the one or more electronic components may include a first electronic component and a second electronic component. The one or more heat pipes may include a first heat pipe and a second heat pipe, wherein the first heat pipe may be coupled to the first electronic component and the second heat pipe may be coupled to the second electronic component. The coupling between the first heat pipe with the first electronic component and the coupling between the second heat pipe with the second electronic component ensures that heat is efficiently transferred from the respective electronic component to the heat exchanger unit, i.e. that heat is removed directly from the corresponding source, thereby improving the overall thermal management of the intraoral scanning device. Advantageously, heat pipes are flexible heat transferring units, as they are effective thermal conductors and their size can easily be adapted and adjusted to the size of the intraoral scanning device.

The intraoral scanning device may comprise a heat dissipation unit that is thermally connected to the one or more electronic units and to the at least one heat transferring unit, and the at least one heat transferring unit may be permanent mounted to the heat dissipation unit. The overheating of the one or more electronic units may cause a drop in the efficiency of said one or more electronic units, e.g. due to throttling, and the overheating may also result in an irreversible damage of the one or more electronic units. Advantageously, the heat dissipation unit ensures that heat is efficiently removed from the one or more electronic units and transferred to the at least one heat transferring unit, preventing the creation of hotspots among the one or more electronic units and therefore ensuring the maximal efficiency of said units.

In one other aspect, there is provided herein an intraoral scanning device configured to perform a scanning of a dental object inside an oral cavity. The intraoral scanning device may comprise one or more electronic units. The intraoral scanning device may comprise a cooling system configured to remove heat from the one or more electronic units. The cooling system may comprise a heat exchanger unit and a flow generating unit. At least a part of the flow generating unit may be covered by the heat exchanger unit. The intraoral scanning device may comprise an air inlet and an air outlet. The flow generating unit may be configured to move air between the air inlet, the air outlet and through the heat exchanger unit. The cooling system may be arranged in vicinity to a rear end of the intraoral scanning device. Thus, the air sucked from the surrounding of the intraoral scanning device into the heat exchanger unit through the air inlet does not encounter any heated component of said device before passing through the heat exchanger unit, thereby enhancing the cooling efficiency of the intraoral scanning device.

The cooling system may be arranged such that a distance must be the shortest possible between the cooling system and the air inlet and the air outlet, respectively. Thus, the air sucked through the air inlet reaches the heat exchanger unit after travelling a distance of less than 1.5 cm, and the air blown out from the heat exchanger unit reaches the air outlet after travelling a distance of less than 1.5 cm. Thus, the heat exchanger unit may include a heatsink with fins arranged on at least two opposite sides of the heatsink, and wherein a distance between the fins and to either the air inlet or air outlet is less than 1.5 cm. This is an advantage, as the distance travelled by the air flow is not large enough to allow for the development of turbulences along the outlet air flow. Furthermore, limiting the distance of the air flowing inside the intraoral scanning device reduces the heating of the flowing air by the heat generated by the battery unit or the one or more electronic units.

The air inlet and the air outlet may be arranged in an end-facet of the intraoral scanning device, wherein the end-facet may be arranged at the rear end of the intraoral scanning device. This ensures maximal hygiene of said device, as the holes for the air inlet and air outlet are placed the farthest possible from the oral cavity of the patient. Furthermore, it prevents the user from being able to close the air inlets and outlets with his/hers hand when holding the intraoral scanning device in the hand.

The air inlet and the air outlet may be separated by the cooling system. Therefore, the air flow does not need to turn and/or change direction when going from the air inlet to the air outlet, i.e. air is sucked into the heat exchanger unit from a first lateral facet of the heat exchanger unit and air is blown out from a second lateral facet of the heat exchanger unit, wherein the second lateral facet is opposite to the first lateral facet. This is an advantage, as the air flow does not encounter any surface of the housing of the intraoral scanning device when going from the heat exchanger unit to the air outlet, thereby preventing the development of turbulences along the outlet air flow. Such an arrangement of the air inlet and the air outlet relative to the cooling system may be the preferred one to optimize the cooling efficiency of the cooling system of the intraoral scanning device.

In another aspect, there is provided herein an intraoral scanning device configured to perform a scanning of a dental object inside an oral cavity. The device may comprise a housing. The housing may comprise one or more electronic units. The housing may further comprise a cooling system configured to remove heat from the one or more electronic units. The cooling system may comprise a heat exchanger unit and a flow generating unit. At least a part of the flow generating unit may be covered by the heat exchanger unit. The housing may comprise one or more processors that are configured to control the flow generating unit such that a temperature of an exterior surface of the housing is between 40 and 50 degrees Celsius. The intraoral scanning device may further include a tip housing attached to the housing, wherein the tip housing includes at least a window. The housing of the intraoral scanning device may be a combination of the first housing and the second housing, or the housing may be either the first housing or the second housing, wherein by first housing and by second housing it is intended the same as described above. Furthermore, the tip housing may be either detachable or permanently mounted to the housing of the intraoral scanning device. Thus, the tip housing corresponds to the part of the intraoral scanning device that is in contact with the oral cavity of the patient, whereas the housing corresponds to the part of the intraoral scanning device which is held in the user's hand to perform the scanning. Advantageously, the one or more processors ensure that the temperature of the exterior surface of the housing of the intraoral scanning device does not exceed the standards imposed by the safety regulations of medical devices, said standards regarding the temperature of the exterior surface of the housing of medical devices.

The intraoral scanning device disclosed herein is therefore safer than current handheld intraoral scanning devices for the user performing the scanning.

In another aspect, there is provided herein an intraoral scanning device configured to perform a scanning of a dental object inside an oral cavity. The intraoral scanning device may comprise one or more electronic units. The intraoral scanning device may further comprise a cooling system configured to remove heat from the one or more electronic units. The cooling system may comprise a heat exchanger unit and a flow generating unit. At least a part of the flow generating unit may be covered by the heat exchanger unit. The intraoral scanning system may further comprise an air inlet and an air outlet. The air inlet and the air outlet may be separated by the heat exchanger. The air inlet and air outlet may be arranged distantly to a tip end of the intraoral scanning device, and the tip end may comprise a scanning window. The flow generating unit may be configured to move air between the air inlet and the air outlet and through the heat exchanger unit in a direction mainly perpendicular to a longitudinal axis of the intraoral scanning device. Advantageously, the air inlet and air outlet are distantly arranged from the oral cavity of a patient when the tip end is inserted into said oral cavity during a scanning session. Thus, air which is blown out of the intraoral scanning device from the air outlet after passing through the heat exchanger does not encounter the oral cavity of the patient, ensuring that standards imposed by the safety regulations of medical devices are respected.

### Brief description of the drawings

Aspects of the present disclosure may be understood from the following detailed description alongside the following drawings. The drawings are schematic and simplified for the sake of clarity, and they are intended to show details to improve the understanding of the claims, whereas other details may be left out. Throughout the description and the drawings, the same reference numbers are used for identical or corresponding parts. The individual features of each aspect may each be combined with any or all features of the other aspects. These and other aspects, features and/or technical effects will be apparent from and elucidated with reference to the illustrations described hereinafter in which:
Fig. 1 illustrates a cross-sectional top view of an example of an intraoral scanning device according to the present disclosure;
Figs. 2a and 2b illustrate cross-sectional views of examples of an intraoral scanning device according to the present disclosure;
Figs. 3a, 3b, 3c, and 3d illustrate a cross-sectional side view of examples of an intraoral scanning device according to the present disclosure;
Fig. 4 illustrates an example of a block diagram of an intraoral scanning device according to the present disclosure;
Figs. 5a, 5b, 5c, 5d and 5e illustrate an exploded perspective view of examples of a heat exchanger unit, a fane unit and a corresponding battery unit; and
Fig. 6 illustrates examples of a temperature measured along an exterior surface of a housing of a first intraoral scanning device and of a second intraoral scanning device, wherein the second intraoral scanning device is an example of an intraoral scanning device according to the present disclosure.

### Detailed description of the drawings

The following detailed description, in connection with the appended drawings, is intended as a description of various configurations of the intraoral scanning device disclosed herein. The detailed description includes specific details to provide a thorough understanding of various concepts. However, it will be clear to a person skilled in the art that these concepts may be practiced without these specific details. Several aspects of the devices are described by various units, modules, components, and so forth (collectively defined to as "elements").

The intraoral scanning device may be configured to acquire surface information of a dental object within the oral cavity of a patient by operated by projecting a pattern and translating a focus plane along an optical axis of the scanning device and capturing a plurality of two-dimensional (2D) images at different focus plane positions, such that each series of captured 2D images corresponding to each focus plane forms a stack of 2D images. The focus plane position may be shifted along the optical axis of the scanning system, such that 2D images captured at a number of focus plane positions along the optical axis form said stack of 2D images for a given view of the object, i.e. for a given arrangement of the intraoral scanning device relative to the object. Said stack of 2D images may be referred to herein as a "sub-scan". After moving the intraoral scanning device relative to the object or imaging the object at a different view, a new sub-scan for that view may be captured, i.e. a plurality of sub-scans is captured by the intraoral scanning device during the live scanning session. The depth information may be estimated based on a focus measure, such that 3D information relative to the surface of the scanned dental object can be obtained from the sub-scans. Another example of an intraoral scanning device is an intraoral scanning device based on triangulation, in which a time varying pattern is projected onto the dental object by means of a projector unit and a sequence of images of the different pattern configurations are acquired by one or more cameras located at an angle relative to the projector unit.

The intraoral scanning device may be a handheld intraoral scanner that is configured to be handled by the hand of a user during the scanning of a patient. A housing of the intraoral scanning device may be designed in an ergonomic manner, such that a single hand of the user is able to hold the scanning device during a scanning session.

The process of obtaining surface information of the dental object to be scanned in real-time may require the intraoral scanning device to illuminate said surface and acquire a high number of 2D images. Typically, a high-speed camera may be used with a framerate of 300-2000 2D images per second, wherein the exact value depends on the technology and 2D image resolution employed by the intraoral scanning device. Thus, a high amount of image data may be handled by the intraoral scanning device, wherein the intraoral scanning device may be either configured to send the acquired image data to an external computer system for the processing of said image data or the intraoral scanning device may be configured to process the image data before sending the processed image data to the external computer system for further processing. In all the above-mentioned configurations, one or more electronic units are needed within the intraoral scanning device to operate with a high workload.

The one or more electronic units may comprise at least one projector unit configured to generate an illumination pattern and to project said pattern onto the dental object to be scanned during a scanning session. The projector unit(s) preferably comprises at least one light source, a mask having a spatial pattern, and one or more lenses such as collimation lenses or projection lenses. The light source may be configured to generate light of a single wavelength or a combination of wavelengths (mono- or polychromatic). A light source configured to produce light comprising different wavelengths, e.g. white light, may be used to produce polychromatic light. Alternatively, the projector unit(s) may comprise one or more light sources individually producing light of different wavelengths that may be combined to form light comprising the different wavelengths, e.g. one or more Light Emitting Diode (LED) units, wherein each LED unit is configured to produce light of different wavelengths, such as red, green and blue, which may be combined to form white light. The intraoral scanning device may further or alternatively comprise a light source configured for exciting fluorescent material of the teeth to obtain fluorescence data from the teeth, e.g. by producing a narrow range of wavelengths to be projected onto the dental objects to be scanned. The light source(s) may be configured to produce infrared (IR) light, and/or ultraviolet (UV) light. The projector unit(s) may be a Digital Light Processing (DLP) projector that uses a micro-mirror array for generating a time varying pattern, or the projector unit(s) may be a diffractive optical element (DOF), or a back-lit mask projector comprising a light source that is placed behind a mask that has a special pattern, such that the light projected onto the dental object is patterned. The back-lit mask projector may comprise a collimation lens for collimating the light from the light source, said collimation lens being placed between the light source and the mask. The mask may have a checkerboard pattern, such that the generated illumination pattern is a checkerboard pattern. Alternatively, the mask may feature other patterns such as lines or dots, etc.

The intraoral scanning device may comprise an image sensor that is configured to capture light that is reflected from the dental object. The image sensor may be configured to generate a plurality of images based on the incoming light received from the illuminated dental object. The image sensor may be a high-speed image sensor, e.g. an image sensor that is configured to acquire images with an exposure of less than 1/1000 second or frame rate larger than 250 frames per second (fps). For example, the image sensor may be a monochrome sensor including a color filter array such as a Bayer filter and/or additional filters that may be configured to substantially remove one or more color components from the reflected light and retain only the remaining non-removed components, prior converting the reflected light into an electrical signal. For example, said additional filters may be used to remove a part of a white light spectrum, such as a blue component, and retain only the red and green components from a light signal that is generated in response to exciting fluorescent material of the teeth.

The intraoral scanning device preferably further comprises one or more processors configured to generate scan data (such as intraoral scan data) by processing the 2D images acquired by the intraoral scanning device. As an example, the one or more processors may comprise a Field-programmable gate array (FPGA) and/or an Advanced RISC Machines (ARM) processor located on the scanning device. The scan data comprises information relating to the dental object. The scan data may comprise any of: 2D images, 3D point clouds, depth data, texture data, intensity data, color data, and/or combinations thereof. In one example, the scan data may comprise one or more point clouds, wherein each point cloud comprises a set of 3D points describing the dental object. In another example, the scan data may comprise images, each image comprising image data, e.g. described by image coordinates and a timestamp (x, y, t), wherein depth information can be inferred from the timestamp. The 2D images acquired by the image sensor may be provided as input to the one or more processors, which process the 2D images to generate scan data. The processing of the 2D images may comprise the step of determining which part of each of the 2D images are in focus, in order to deduce/generate depth information from the images. The internal depth information may be used to generate 3D point clouds comprising a set of 3D points in space, e.g., described by cartesian coordinates (x, y, z). The 3D point clouds may be generated by the one or more processors or by another processing unit, e.g. a processing unit comprised within a computer system that is configured to receive, either wirelessly or through wired connection, data from the intraoral scanning device. Each 2D/3D point may further comprise a timestamp that indicates when the 2D/3D point was recorded, i.e., from which image in the stack of 2D images the point originates. The timestamp is correlated with the z-coordinate of the 3D points, i.e., the z-coordinate may be inferred from the timestamp. Accordingly, the output of the processor is the scan data, and the scan data may comprise image data and/or depth data, e.g. described by image coordinates and a timestamp (x, y, t) or alternatively described as (x, y, z). The intraoral scanning device may be configured to acquire and transmit other types of data in addition to the scan data. Examples of data include 3D information, texture information such as IR images, fluorescence images, reflectance color images, x-ray images, and/or combinations thereof.

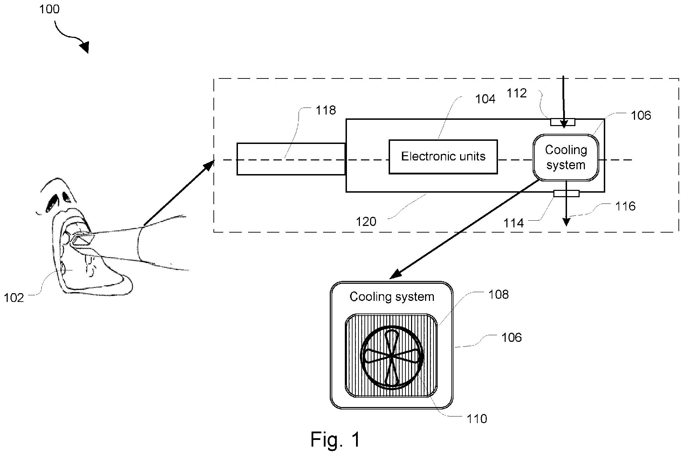

Fig. 1 illustrates a cross-sectional top view of an example of an intraoral scanning device 100 according to the present disclosure. The intraoral scanning device is configured to acquire real-time data comprising surface information of a three-dimensional (3D) dental object 102 in an oral cavity of a patient. The dental object may be any portion of the patient's oral cavity, such as dentition, gingiva, hard palate, soft palate, and so forth. The data relative to said dental object may be acquired during a live scanning session of the patient's oral cavity, during which a user, such as a dentist and/or a dental assistant and/or any other user with dental knowledge, may scan by means of the intraoral scanning device 100 the dental object 102, while the patient is sitting in a chair, e.g. in a dental studio. The intraoral scanning device 100 comprises one or more electronic units 104, wherein the one or more electronic units 104 may be configured to perform a number of functions, including, but not limited to, acquiring data relative to the dental object 102, and/or processing said data, and/or transmitting said data to an external device and/or system, e.g. a computer system, and so forth. While the intraoral scanning device 100 is in use, the one or more electronic units 104 dissipate power into heat, thereby causing the development of hotspots along the intraoral scanning device 100. It is therefore crucial to ensure efficient thermal management within the intraoral scanning device 100 to guarantee the safety of the user performing the scanning of the dental object 102. Thus, the intraoral scanning device 100 comprises an integrated cooling system 106 that is configured to remove from the intraoral scanning device 100 the heat generated by the one or more electronic units 104. The cooling system 106 comprises a heat exchanger unit 108 and a flow generating unit 110, wherein the flow generating unit 110 is arranged in such a way that at least a part of the flow generating unit 110 is covered by the heat exchanger unit 108. In the example illustrated in Fig. 1, the flow generating unit 110 is completely covered by the heat exchanger unit 108. However, any other arrangement of the flow generating unit 110 in which said flow generating unit 110 is at least partially covered by the heat exchanger unit 108 is possible. Additionally, in the example illustrated in Fig. 1, the flow generating unit 110 is a fane unit. The intraoral scanning device 100 further comprises an air inlet 112 and an air outlet 114, wherein the air inlet 112 and the air outlet 114 are separated by the heat exchanger unit 108. The fane unit 110 is configured to move air between the air inlet 112, the air outlet 114 and through the heat exchanger unit 108 in a direction 116 that is mainly perpendicular to a longitudinal axis 118 of the device 100. Therefore, air is sucked into the heat exchanger unit 108 from one lateral facet of said heat exchanger unit 108 through the air inlet 112, and air is blown out from an opposite lateral facet of the heat exchanger unit 108 through the air outlet 114. In this example of the intraoral scanning device 100, the outlet air flow does not encounter any surface of a housing 120 of the intraoral scanning device 100, therefore the development of a pressure drop across the heat exchanger unit 108 and the development of turbulences in the outlet air flow are prevented in the device 100.

For the sake of simplicity, the flow generating unit 110 will be illustrated herein and referred to herein as the "fane unit 110", however it will be appreciated that the fane unit 110 could be replaced with a blower, a solid stage cooler, a piezo cooler and so forth.

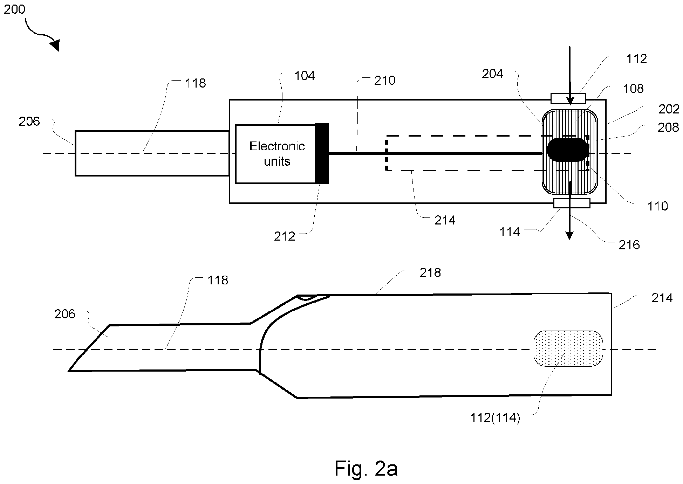

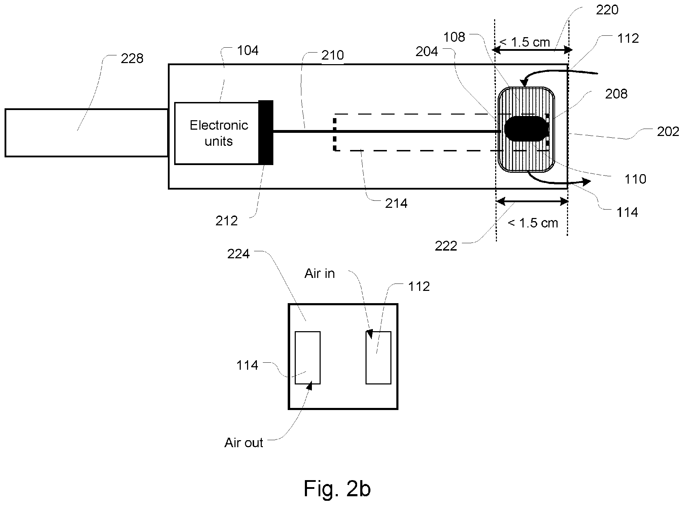

Figs. 2a and 2b illustrate cross-sectional views of examples of an intraoral scanning device 200 according to the present disclosure. In particular, the top panels of Figs. 2a and 2b illustrate a cross-sectional top view of two examples of the intraoral scanning device 200. The intraoral scanning device 200 comprises one or more electronic units 104 that generate heat while the intraoral scanning device 200 is in use, wherein said heat is generated due to the dissipation of power from the one or more electronic units 104. The one or more electronic units 104 may be configured to perform a number of functions, including, but not limited to, acquiring data relative to a 3D dental object within the oral cavity of a patient, processing said data relative to the 3D dental object, transmitting said data relative to the 3D dental object to an external device and/or system, such as a computer system, and so forth. The intraoral scanning device 200 further comprises a heat exchanger unit 108 and a fane unit 110, wherein the fane unit 110 is arranged such that at least a part of the fane unit 110 is covered by the heat exchanger unit 108. In the examples illustrated in Figs. 2a and 2b, the fane unit 110 is completely covered by the heat exchanger unit 108. The heat exchanger unit 108 and the fane unit 110 together form a cooling system of the intraoral scanning device 200, wherein said cooling system is configured to remove from the intraoral scanning device 200 the heat generated by the one or more electronic units 104. In both the examples illustrated in Figs. 2a and 2b, the heat exchanger unit 108 is arranged in vicinity to a rear end 202 of the intraoral scanning device 200. Additionally, the heat exchanger unit 108 comprises two sides: a first side 204 that faces a tip end 206 of the intraoral scanning device 200, and a second side 208 that faces the rear end 202 of the intraoral scanning device 200. The heat exchanger unit 108 is thermally coupled to the one or more electronic units 104 by means of at least one heat transferring unit 210, wherein the at least one heat transferring unit 210 is configured to transfer heat from the one or more electronic units 104 to the heat exchanger unit 108. In one example, the at least one heat transferring unit 210 comprises one or more heat pipes, wherein the one or more heat pipes comprise a working fluid, such as a liquid. In this example, the one or more heat pipes may comprise a first heat pipe and a second heat pipe, wherein the first heat pipe may be thermally coupled to a first electronic component and the second heat pipe may be thermally coupled to a second electronic component, and wherein the first electronic component and the second electronic component are comprised within the one or more electronic components 104. The coupling between the first heat pipe with the first electronic unit and the coupling between the second heat pipe and the second electronic unit may improve the efficiency of heat removal from the one or more electronic units 104, thereby preventing the overheating of said one or more electronic units 104 and maximizing the power efficiency of the one or more electronic units 104. In another example, the at least one heat transferring unit 210 is a thermal plate connecting the one or more electronic units 104 to the heat exchanger unit 108. In yet another example, the at least one heat transferring unit 210 is a vapor chamber connecting the one or more electronic units 104 to the heat exchanger unit 108. The use of a thermal plate and/or of a vapor chamber may be advantageous, for example, to maximize the area over which the heat removed from the one or more electronic units 104 is spread. The intraoral scanning device 200 may further comprise a heat dissipation unit 212 that is thermally connected to the one or more electronic units 104 and to the heat transferring unit 210. For example, the heat dissipation unit 212 may be a thermally conductive plate which is configured to maximize the heat transfer efficiency from the one or more electronic units 104 to the heat transferring unit 210. The heat transferring unit 210 is permanent mounted to the heat dissipation unit 212, and said heat transferring unit 212 is permanent or detachable mounted to the first side 204 of the heat exchanger unit 108. The intraoral scanning device 200 further comprises a battery unit 214 that is configured to power the fane unit 110 through at least one electrical connection or through at least one inductive connection. For example, the electrical connection may be realized by means of at least one electrically conductive wire which connects the fane unit 110 to the battery unit 214. In one example, the inductive connection may be instead realized by means of an electromagnetic field that is generated within the power unit of the battery, e.g. through an alternating current flowing through an inductor (such as a transmitter coil) comprised within the battery unit, and is received by the fane unit, e.g. through a receiver coil. The fane unit 110 is permanent or detachable mounted to a rear end of the battery unit 214, and the battery unit 214 is configured to slide through the heat exchanger unit 108 until at least a part of the fane unit 110 is covered by the heat exchanger unit 108. The intraoral scanning device 200 further comprises an air inlet 112 and an air outlet 114.

Referring to the example illustrated in Fig. 2a, the air inlet 112 and the air outlet 114 are arranged in proximity to the rear end 202 of the intraoral scanning device 200 and are separated by the heat exchanger unit 108. In this example, the fane unit 110 is configured to move air between the air inlet 112, the air outlet 114 and through the heat exchanger unit 108 in a direction 216 mainly perpendicular to a longitudinal axis 118 of the intraoral scanning device 200, wherein the longitudinal axis 118 extends between the tip end 206 and the rear end 202 of the intraoral scanning device 200. Illustrated at the bottom of Fig. 2a is a cross-sectional side view of the intraoral scanning device 200. The air inlet 112 and the air outlet 114 may be realized by means of two grids which are arranged on two opposite lateral facets of a housing 218 of the intraoral scanning device 200, such that the air inlet 112 and the air outlet 114 are separated by the heat exchanger unit 108. Referring to the bottom of Fig. 2a, one grid, e.g. the air inlet 112 (or the air outlet 114), is shown on the illustrated lateral facet of the intraoral scanning device 200, and an analogous grid, e.g. the air outlet 114 (or the air inlet), is arranged on the opposite side of the device 200 (not shown), such that air is sucked in from one lateral facet of the heat exchanger unit 108 and air is blown out from an opposite lateral facet of the heat exchanger unit 108.

The top panel of Fig. 2b illustrates a cross-sectional top view of an example of the intraoral scanning device 200 alternative to the example illustrated in Fig. 2a. In this example, the air inlet 112 and the air outlet 114 are arranged such that a distance 220 of less than 1.5 cm is achieved between the heat exchanger unit 108 and the air inlet 112, and a distance 222 of less than 1.5 cm is achieved between the heat exchanger unit 108 and the air outlet 114. The bottom panel of Fig. 2b illustrates a cross-sectional back view of the intraoral scanning device 200 according to the example illustrated in the top panel of Fig. 2b. The air inlet 112 and the air outlet 114 are arranged in an end-facet 224 of the intraoral scanning device 200, wherein the end-facet 224 is located in proximity of the rear end 202 of the intraoral scanning device 200.

In order to prevent the development of air turbulences across the heat exchanger unit 108, the configuration of the intraoral scanning device 200 as illustrated in Fig. 2a may be preferable, since in this example the outlet air 114 does not encounter any surface of the outer housing shell 218 of the intraoral scanning device 200. However, it will be appreciated by those skilled in the art that the configuration of the intraoral scanning device 200 as illustrated in Fig. 2b is also advantageous, as the air flow travels a distance less than 1.5 cm between the air inlet 112 and the heat exchanger unit 108, and a distance of less than 1.5 cm between the heat exchanger unit 108 and the air outlet 114, such that the flow resistance is reduced, thereby preventing the development of air turbulences.

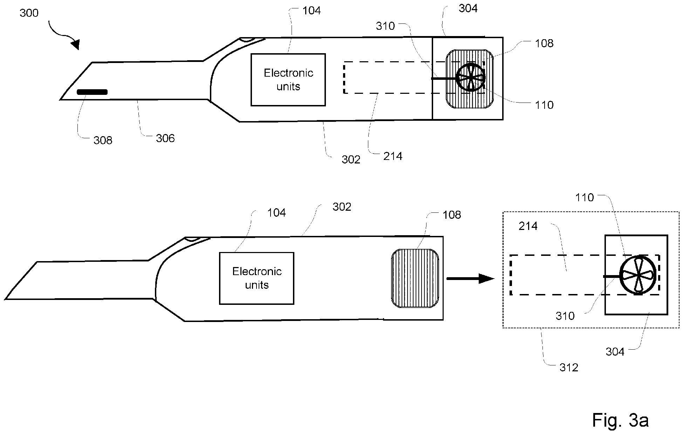

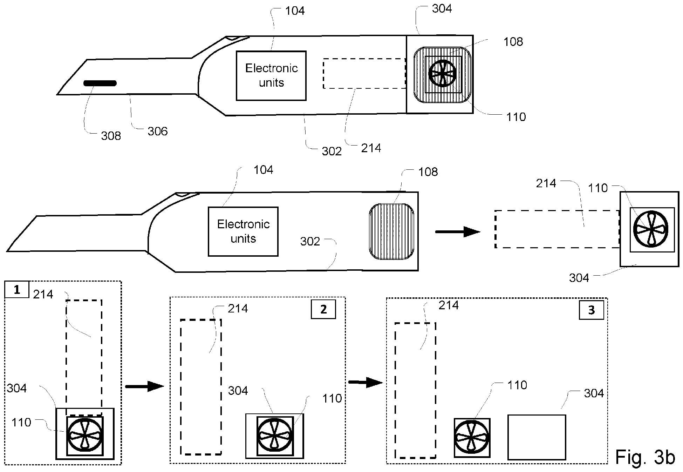

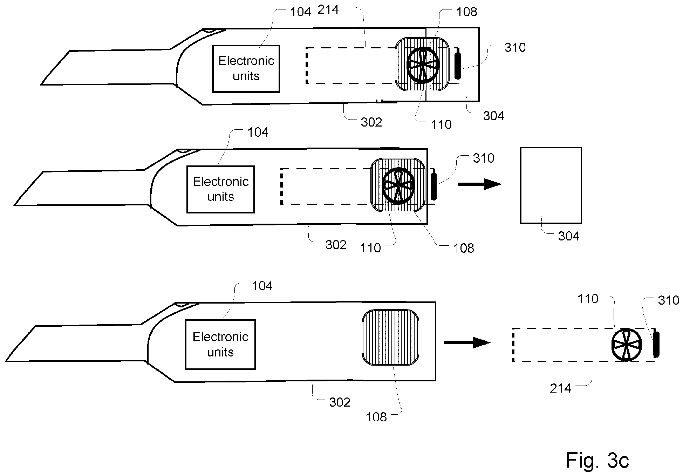

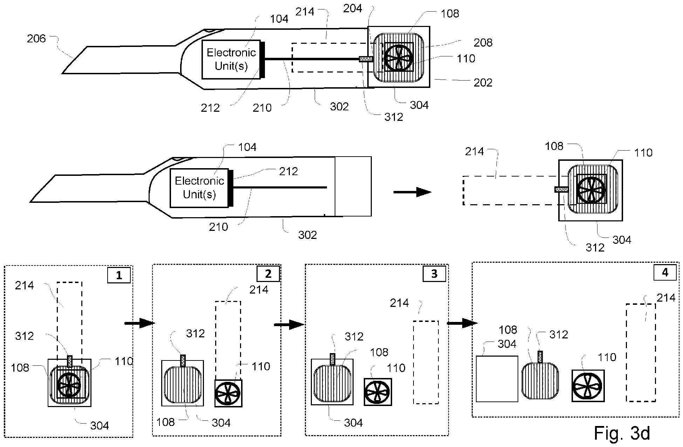

Fig. 3a, 3b, 3c and 3d illustrate a cross-sectional side view of examples of intraoral scanning devices according to the present disclosure. Referring to Figs. 3a, 3b, 3c and 3d, the intraoral scanning device 300 comprises a first housing 304 and a second housing 302, wherein the first housing 304 is detachable mounted to the second housing 302. The first housing 304, or the second housing 302, or a combination thereof may be referred to herein as a "housing" of the intraoral scanning device 300. Referring to the examples illustrated in Figs. 3a, 3b, 3c and 3d, the housing of the intraoral scanning device 300 is a combination of the first housing 304 and of the second housing 302. The intraoral scanning device 300 may further comprise a tip part 306 that is attached to the housing of the intraoral scanning device 300, wherein the tip part 306 comprises at least one mirror 308. The second housing 302 comprises one or more electronic units 104 that generate heat while the device is in use, and said electronic units 104 are permanent mounted to the second housing 302 of the intraoral scanning device 300. The intraoral scanning device 300 further comprises a heat exchanger unit 108 and a fane unit 110, such that the heat exchanger unit 108 and the fane unit 110 together form a cooling system of the intraoral scanning device 300 that is configured to remove heat from the one or more electronic units 104, i.e. said cooling system is responsible for the thermal management of the intraoral scanning device 300. The intraoral scanning device 300 further comprises a battery unit 214 that is configured to power the fane unit 110 through at least one connection.

Referring to Fig. 3a, the battery unit 214 is configured to power the fane unit 110 through at least one electric connection 310. Such an electric connection 310 may be easily and cheaply implemented in current intraoral scanning devices, and it also guarantees stable powering of the fane unit 110 from the battery unit 214. For example, the electric connection 310 may be realized by means of at least one electrically conductive wire that connects the fane unit 110 to a power unit of the battery unit 214. Therefore, in the example illustrated in Fig. 3a the fane unit 110 is permanently mounted to the battery unit 214, such that the battery unit 214 together with the fane unit 110 form a single unit 312 that may be referred to herein as "fan-integrated battery". The fan-integrated battery 312 is configured to slide through the heat exchanger unit 108 until at least a part of the fane unit 110 is covered by the heat exchanger unit 108. Thus, the fan-integrated battery 312 can be pulled out from the intraoral scanning device 300, for example to recharge and/or to replace said fan-integrated battery 312, as illustrated in the bottom panel of Fig. 3a. Additionally, as shown in the bottom panel of Fig. 3a, the fan-integrated battery 312 is permanently mounted to the first housing 304 of the intraoral scanning device 300, such that when the first housing 304 is detached from the second housing 302, the fan-integrated battery 312 is maintained in the first housing 304, whereas the electronic units 104 and the heat exchanger unit 108 are maintained in the second housing 302 of the intraoral scanning device 300.

Fig. 3b illustrates a cross-sectional side view of an example of the intraoral scanning device 300 alternative to the example illustrated in Fig. 3a. In the example illustrated in Fig. 3b, the fane unit 110 is detachable mounted to the battery unit 214, e.g. by means of a magnetic or snap couple or by means of a Universal Serial Bus (USB) couple. For example, the fane unit 110 may be powered by the battery unit 214 through an inductive connection, which may be realized by means of an electromagnetic field. Said electromagnetic field may be generated within the power unit of the battery unit 214, for example, through an alternating current flowing through an inductor (such as a transmitter coil) comprised within the battery unit 214, and the electromagnetic field may be received by the fane unit 110, e.g. through a receiver coil. In another example, the fane unit 110 may be powered by the battery unit 214 through an electrical connection, which may be realized by means of a USB connection. Said USB connection may be realized upon plugging a USB plug comprised in the fane unit 110 into a USB port comprised in the battery unit 214. The battery unit 214 and the fane unit 110 are mounted to the first housing 304 of the intraoral scanning device 300, such that when the first housing 304 is detached from the second housing 302, the fane unit 110 and the battery unit 214 are maintained in the first hosing 304, as illustrated in the middle panel of Fig. 3b and in panel number 1 in the bottom of Fig. 3b. In one example, the battery unit 214 is detachable from the first housing 304, whereas the fane unit 110 is permanent mounted to the first housing 304, as illustrated in the panel number 2 in the bottom of Fig. 3b. In another example, both the battery 214 and the fane unit 110 are detachable mounted to the first housing 308, as illustrated in the panel number 3 in the bottom of Fig. 3b.

Fig. 3c illustrates a cross-sectional side view of an example of the intraoral scanning device 300 alternative to the examples illustrated in Figs. 3a and 3b. In this example, the battery unit 214 and the fane unit 110 are detachable mounted to the first housing 304 of the intraoral scanning device 300, such that when the first housing 304 is detached from the first housing 302, the battery unit 214 and the fane unit 110 are maintained in the second housing 302, as illustrated in the middle panel of Fig. 3c. In this example, the fane unit 110 is permanent mounted to the battery unit 214, and the battery unit 214 comprises an extractor 310 that is configured to pull out the battery unit 214 and the fane unit 110 from the intraoral scanner device 300, upon a user of the intraoral scanner device 300 pulling the extractor 310, as illustrated in the bottom panel of Fig. 3c. In another example (not shown in Fig. 3c), the fane unit 110 is detachable mounted to the battery unit 214, and the extractor 310 is mounted to the fane unit 110, such that the fane unit 110 and the battery unit 214 can be pulled out from the second housing 302 by means of the extractor 310. In this second example, the fane unit 110 can be detached by the battery unit 214 by means of a magnetic or snap couple after being extracted by the intraoral scanning device 300 by means of the extractor 310.

Fig. 3d illustrates a cross-sectional side view of an example of the intraoral scanning device 300 alternative to the examples illustrated in Figs. 3a, 3b and 3c. The intraoral scanning device 300 comprises a heat exchanger unit 108 that is thermally coupled to one or more electronic units 104 by means of at least one heat transferring unit 210. For example, the at least one heat transferring unit 210 may be at least one heat pipe that comprises a working fluid, wherein said heat pipes(s) is configured to transfer heat from the one or more electronic components 104 to the heat exchanger unit 108. In another example, the at least one heat transferring unit 210 is a thermal plate that is mounted to the one or more electronic units 104 and to the heat exchanger unit 108. The heat exchanger unit 108 comprises two sides: a first side 204 that faces a tip end 206 of the intraoral scanning device 300, and a second side 208 that faces a rear end 202 of the intraoral scanning device 300. The second side 208 is mounted to a first housing 304 of the intraoral scanning device, wherein said first housing 304 is detachable from a second housing 302 of the intraoral scanning device 300, such that when the first housing 304 is detached from the second housing 302 the heat exchanger unit 208 is maintained in the first housing 304. In order maximize the heat transfer efficiency from the one or more electronic units 104 to the at least one heat transferring unit 210, the intraoral scanning device 300 may further comprise a heat dissipation unit 212. In the example shown in Fig. 3d, the heat transferring unit(s) 210 is permanent mounted to the heat dissipation unit 212 and detachable mounted to the first side 204 of the heat exchanger unit 108 by means of a coupling 312. For example, the coupling 312 may be realized by means of a magnetic force, or the coupling 312 may be a snap coupling. Thus, when the heat exchanger unit 108 is pulled out from the intraoral scanning device 300 by means of the first housing 304, the one or more electronic units 104, the at least one heat transferring unit 210 and the heat dissipation unit 212 are maintained in the second housing 302, as illustrated in the middle panel of Fig. 3d. Additionally, the battery unit 214 and the fane unit 110 are mounted to the first housing 304, such that when the first housing 304 is detached from the second housing 302 the battery unit 214 and the fane unit 110 are also maintained in the first housing 304, as illustrated in the middle panel of Fig. 3d. In one example, the battery unit 214, the fane unit 110 and the heat exchanger unit 108 are permanent mounted to the first housing 304, as illustrated in panel number 1 in the bottom of Fig. 3d. In another example, the battery unit 214 and the fane unit 110 are detachable mounted to the first housing 304, such that the battery unit 214 and the fane unit 110 can be pulled out from the first housing 304, whereas the heat exchanger unit 108 is permanent mounted to the first housing 304, as illustrated in panel number 2 in the bottom of Fig. 3d. In yet another example, the fane unit is detachable mounted to the battery unit 214, such that, after pulling the battery unit 214 and the fane unit 110 out of the first housing 304, the fane unit 110 can be detached from the battery unit 214, as illustrated in panel number 3 in the bottom of Fig. 3d. In yet another example, the heat exchanger unit 108 is also detachable mounted to the first housing 304, such that the heat exchanger unit 108 can be pulled out from the first housing 304, as illustrated in panel 4 in the bottom of Fig. 3d.

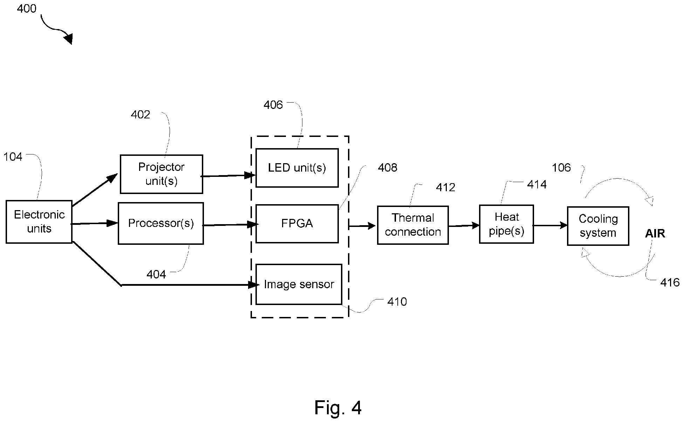

Fig. 4 illustrates an example of a block diagram of an intraoral scanning device 400 according to the present disclosure. The intraoral scanning device 400 comprises one or more electronic units 104, one thermal connection 412, one or more heat pipes 414 and a cooling system 106. The one or more electronic units 104 comprise at least one projector unit 404, one or more processor units 406 and an image sensor 410. The projector unit(s) 402 is configured to project light onto a dental object in an oral cavity by means of at least one light source. In the example shown in Fig. 4, the at least one light source is at least one LED unit 406 that is configured to generate light of a single wavelength and/or light that is a combination of wavelengths. The one or more processor units 404 are configured to process, partially or completely, the data acquired from the intraoral scanning device 400, wherein said data describe a surface of a dental object within an oral cavity of a patient. For example, the intraoral scanning device 400 may be configured to acquire 2D images describing at least a part of a surface of the dental object, and the one or more processors 404 may be configured to generate scan data based on the 2D images acquired by the intraoral scanning device 400. The one or more processors 404 comprise a field-programmable gate array (FPGA) 408, however in another example (not shown in Fig. 4) the one or more processors 406 may further or alternatively comprise an advanced RISC machine (ARM). Finally, the image sensor 410 is configured to detect the light reflected from the scanned dental object and to convert the detected light into an electric signal. The at least one LED unit 406, the FPGA 408 and the image sensor 410 constitute the main sources of heat for the intraoral scanning device 400, as these units dissipate a large part of their power into heat when the intraoral scanning device 400 is in use. In order to remove said heat from the intraoral scanning device 400, the main sources of heat are thermally coupled with the cooling system 106. In the example illustrated in Fig. 4, the thermal coupling is achieved by means of a thermal connection 412 between the sources of heat and one or more heat pipes 414, wherein the thermal connection 412 is configured to couple the main sources of heat to the one or more heat pipes 414, and wherein the one or more heat pipes 414 are configured to transfer heat from the main sources of heat to the cooling system 106. In another example (not shown in Fig. 4), the heat pipes 414 may be replaced by a thermally conductive plate. The cooling system 106 is configured to generate a flow of air 416 between the intraoral scanning device 400 and the surrounding, such that the cooling system 106 sucks air from the surrounding into the intraoral scanning device 400, the air passes through the cooling system 106 and it exchanges heat with the cooling system, i.e. thermal energy is transferred from the cooling system 106 to the flow of air 416, and then heated air is blown out from the intraoral scanning device 400. Thus, the cooling system 106, the thermal connection 412 and the heat pipes 414 contribute to the thermal management of the intraoral scanning device 400.

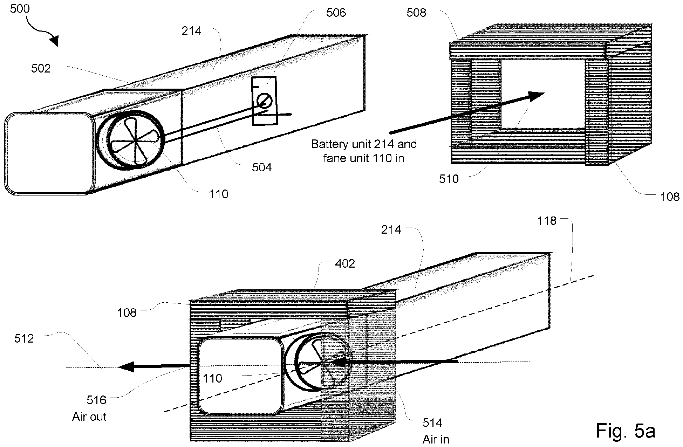

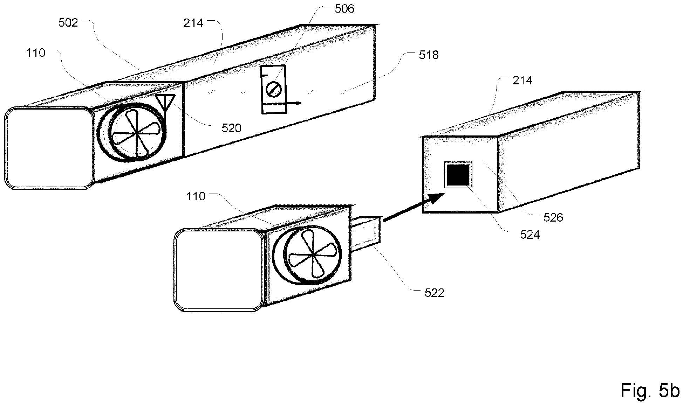

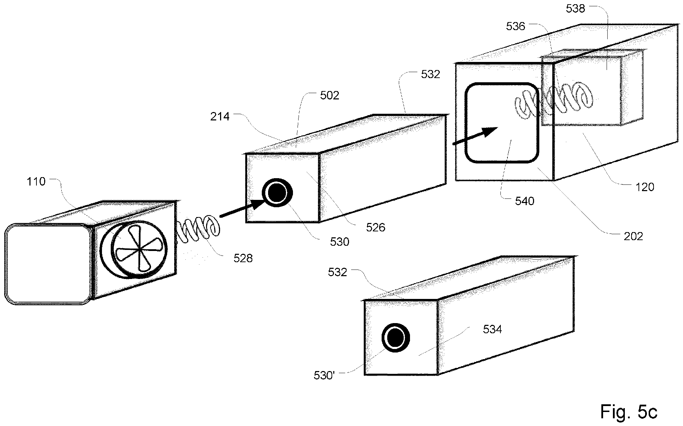

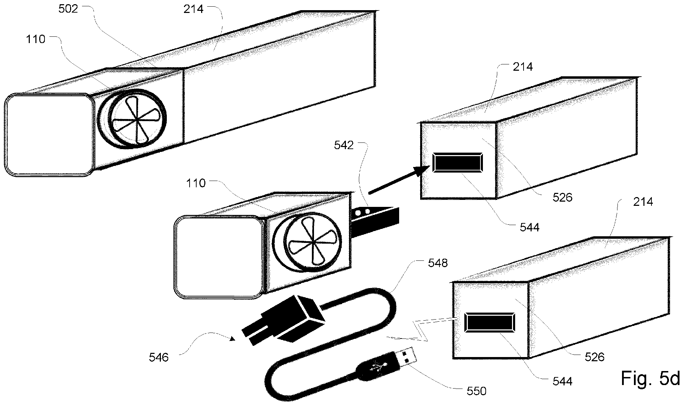

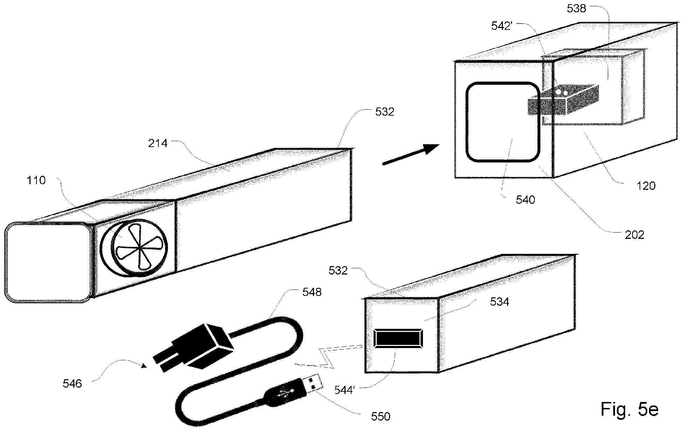

Fig. 5a, 5b, 5c, 5d and 5e illustrate an exploded perspective 500 of examples of a heat exchanger unit 108, a fane unit 110 and a battery unit 214 according to the present disclosure. Referring to Fig. 5a, the battery unit 214 comprises a fane unit 110, and in this example the fane unit 110 is permanent mounted in vicinity to a rear end 502 of the battery unit 214. The battery unit 214 is configured to power the fane unit 110 by means of an electrical connection 504, wherein the electrical connection 504 may be realized by means of one or more electrically conductive wires. Said electrical connection 504 connects the fane unit 110 to a power unit 506 of the battery unit 214. The heat exchanger unit 108 is a heatsink which comprises fins 508, wherein said fins 508 define a cavity 510. The battery unit 214 is configured to slide through the cavity 510 of the heat exchanger unit until at least a part of the fane unit 110 is covered by the heat exchanger unit 214, as illustrated in the bottom panel of Fig. 5a. The fane unit 110 is an axial-flow fan which has an axis of rotation 512, wherein said axis of rotation 512 is perpendicular to a longitudinal axis 118 of an intraoral scanning device as illustrated as reference number 100 in Fig. 1. The fane unit 110 is configured to move air in a direction mainly parallel to its axis of rotation 512. In particular, the fane unit 110 is configured to suck air into the heat exchanger unit 108 from a first lateral facet 514 of the heat exchanger unit 108, let air pass through the heat exchanger unit 108, and then blow air out from a second lateral facet 516 of the heat exchanger unit 108, wherein the second lateral facet 516 is opposite to the first lateral facet 514.

Fig. 5b illustrates an exploded perspective of an example of a battery unit 214 and corresponding fane unit 110 alternative to the example illustrated in Fig. 5a. In this example, the fane unit 110 is detachable mounted to the battery unit 214, i.e. the fane unit 110 can be detached from the battery unit 214 and the fane unit 110 can be re-attached to the battery unit 214. The fane unit 110 may be powered by the battery unit by means of an inductive connection, that may be realized by means of an electromagnetic field 518. For example, the electromagnetic field 518 may be produced through an alternating current flowing through an inductor (such as a transmitter coil) comprised within a power unit 506 of the battery unit 214. The electromagnetic field 518 may be then received by the fane unit through a receiver unit 520 comprised within the fane unit 110. In the example illustrated in Fig. 5b, the fane unit 110 comprises a plug 522 which can be inserted into/removed from a socket 524 that is arranged on an end-facet 526 of the battery unit 214, wherein the end-facet 526 of the battery unity is arranged at the rear end 502 of the battery unit 214. Thus, the fane unit 110 is detachable mounted to the battery unit 214 by means of a snap couple. In another example (not shown here), the fane unit 110 may be detachable mounted to the battery unit 214 by means of a magnetic couple.

Fig. 5c illustrates an exploded perspective of yet another example of a battery unit 214 and corresponding fane unit 110 alternative to the examples illustrated in Fig. 5a and in Fig. 5b. In this example, the fane unit 110 is detachable mounted to the battery unit 214, i.e. the fane unit 110 can be detached from the battery unit 214 and the fane unit 110 can be re-attached to the battery unit 214. The fane unit 110 comprises a spring 528 which can be locked into/detached from an anchor point 530 that is placed on an end-facet 526 of the battery unit 214, such that the fane unit 110 can be attached to the battery unit 214 by means of a spring force. A similar second anchor point 530' may be arranged on a front-facet 534 of the battery unit 214, wherein the front-facet is arranged on a front end 532 of the battery unit 214. Thus, the battery unit 214 may be configured to slide into a hole 540 that is arranged on an end-facet 202 of an intraoral scanning device according to the present disclosure (such as the one illustrated as reference number 100 in Fig. 1, and/or the one illustrated as reference number 200 in Fig.2, and/or the one illustrated as reference number 300 in Fig. 3, not shown here), and the battery unit 514 may be configured to slide into the hole 540 until a second spring 536 snaps into the second anchor point 530'. Said second spring 536 may be arranged on an element 538 placed into a housing 120 of the intraoral scanning device according to the present disclosure (not shown here), such that the battery unit 214 is held in place into the housing 120 by a spring force exerted on the rear end 502 and on the front end 532 of the battery unit 214. The element 538 may be, for example, a mainboard of the intraoral scanning device, or a thermally conductive plate as the one shown as reference number 212 in Figs. 2a, 2b and 3d.

Fig. 5d illustrates an exploded view of yet another example of a battery unit 214 and corresponding fane unit 110 alternative to the examples illustrated in Fig. 5a, 5b and 5c. In this example, the fane unit 110 is detachable mounted to the battery unit 214, in particular the fane unit 110 can be detached from the battery unit 214, and the fane unit 110 can be re-attached to the battery unit 214 by means of a connection between a Universal Serial Bus (USB) plug 542 which can be connected to a USB port 544. Accordingly, the fane unit 110 comprises the USB plug 542, for example a USB Type C (USB-C), and the battery unit 214 comprises the USB port 544 which is arranged on an end-facet 526 of the battery unit 214. The USB plug 542 is of the same type as the USB port 544, in other words, if the USB port 544 is a USB-C port, then the USB plug 542 is a USB-C plug configured to be plugged into the USB-C port 544. Thus, the battery unit 214 may be configured to power the fane unit 110 by means of the connection which is realized when the USB plug 542 is plugged into the USB port 544. Advantageously, the USB port 544 may be configured to generate an electrical connection between the battery unit 214 and a charging unit 546, the latter comprising a wire 548 and a USB plug 550 which is configured to be connected to the USB port 544. In other words, the battery unit 214 may be charged upon connecting the battery unit 214 to the charger 546 by means of the USB port 544, wherein the charger 546 may be any charger comprising a USB plug compatible with the USB port 544, e.g. a tablet charger, or a smartphone charger, or a charger of a set of headphones, and so forth. This may be advantageous to reduce the costs of production of the battery unit 214 of the intraoral scanning device according to the present disclosure: first, USB connections can be easily and cheaply implemented in typical intraoral scanning devices; second, any charger unit with a USB plug configured to be connected to the USB port 544 comprised in the battery unit 214 may be used to charge the battery unit 214, i.e. it is not needed to produce and/or buy a specific charger for the battery unit 214.

Fig. 5e illustrates an exploded view of yet another example of a battery unit 214 and corresponding fane unit 110 further or alternative to the examples illustrated in Fig. 5a, 5b, 5c, and 5d. In this example, the fane unit 110 is permanently mounted to the battery unit 214, i.e. a fan-integrated battery is provided. For example, the battery unit 214 may be configured to power the fane unit 110 by means of an electrical connection as the one illustrated as reference number 504 in Fig. 5a. However, in another example (not shown here) the fane unit 110 may be detachable mounted to the battery unit 214, for example the fane unit 110 may be powered by the battery unit 214 by means of an inductive connection realized by means of an electromagnetic field as the one illustrated as reference number 518 in Fig. 5b. The battery unit 214 may comprise a USB port 544', for example as a USB port of type C, which may be arranged in the vicinity of a front end 532 of the battery unit 214, and in particular the USB port 544' may be arranged on a front-facet 534 of the battery unit 214. Thus, the battery unit 214 may be configured to slide into a hole 540 that is arranged on an end-facet 202 of an intraoral scanning device according to the present disclosure (such as the one illustrated as reference number 100 in Fig. 1, and/or the one illustrated as reference number 200 in Fig. 2, and/or the one illustrated as reference number 300 in Fig. 3, not shown here). The battery unit 214 may be configured to slide into the hole 540 until a USB plug 542' arranged on an element 538 placed into a housing 120 of the intraoral scanning device according to the present disclosure is plugged into the USB port 544'. The USB plug 542' is of the same type of the USB port 544', e.g. if the USB port 544' is a USB-C port then the USB plug 542' is a USB-C plug. In other words, the USB plug 542' is compatible with the USB port 544' and the USB plug 542' is configured to be connected to the USB port 544'. Thus, when the battery 214 is inserted into the intraoral scanning device, the battery unit 214 is held in place into the housing 120 by the connection obtained when the USB plug 542' is connected to the USB port 544'. The element 538 may be, for example, a mainboard of the intraoral scanning device, or a thermally conductive plate as the one shown as reference number 212 in Figs. 2a, 2b and 3d. Further, the battery unit 214 may be configured to power one or more electronic units of the intraoral scanning device via the electrical connection generated by means of the connection between the USB port 544' and the USB plug 542'. Advantageously, the USB port 544' may be configured to generate a connection between the battery unit 214 and a charging unit 546, the latter comprising a wire 548 and a USB plug 550 of the same type as the USB port 544' which is configured to be connected to the USB port 544'. In other words, the battery unit 214 may be charged upon connecting the battery unit 214 to the charger 546, i.e. by connecting the USB plug 550 to the USB port 544', wherein the charger 546 may be any charger comprising a USB plug configured to be connected to the USB port 544', e.g. a tablet charger, or a smartphone charger, or a charger of a set of headphones, and so forth. This example may be advantageous to reduce the costs of production of the battery unit 214 of the intraoral scanning device according to the present disclosure: first, USB connections can be easily and cheaply implemented in current intraoral scanning devices; second, any charger unit with a USB plug of the same type as the USB port 544' comprised in the battery unit 214 may be used to charge the battery unit 214, i.e. it is not needed to produce and/or buy a specific charger for the battery unit 214.

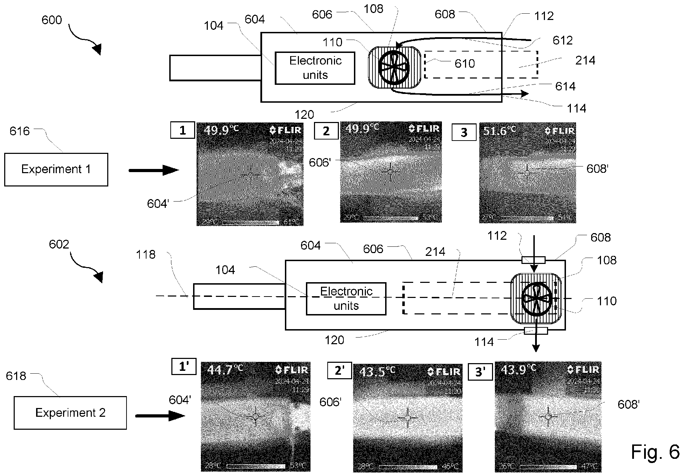

Fig. 6 illustrates examples of a temperature measured along an exterior surface of a housing 120 of a first intraoral scanning device 600 and of a second intraoral scanning device 602, wherein the second intraoral scanning device 602 is an example of intraoral scanning device according to the present disclosure. As shown in the top panel of Fig. 6, the first intraoral scanning device 600 comprises one or more electronic units 104 which generate heat while the intraoral scanning device 600 is in use. The intraoral scanning device 600 further comprises a heat exchanger unit 108 and a fane unit 110, wherein the fane unit 110 is arranged such that at least a part of the fane unit 110 is covered by the heat exchanger unit 108. The heat exchanger unit 108 and the fane unit 110 together form a cooling system of the intraoral scanning device 600 that is configured to remove heat from the one or more electronic units 104. The intraoral scanning device 600 further comprises a battery unit 214 that is configured to slide into the intraoral scanning device 600, e.g. through an aperture/hole placed on an end-facet arranged at a rear end 608 of the intraoral scanning device 600. The heat exchanger unit 108 and the fane unit 110 are arranged in the housing 120 in such a way that, when the battery unit 214 is inserted into the intraoral scanning device 600, a front end 610 of the battery unit 214 is arranged in vicinity to the heat exchanger unit 108 and to the fane unit 110. The intraoral scanning device 600 further comprises an air inlet 112 and an air outlet 114, wherein the air inlet 112 and the air outlet 114 are arranged in vicinity to the rear end 608 of the intraoral scanning device 600 and the air inlet 112 and the air outlet 114 are separated by the battery unit 214. The fane unit 110 is configured to suck air through the air inlet 112, let air pass through the heat exchanger unit 108, and finally blow the air out through the air outlet 114. In particular, the inlet air flow moves along a first direction in a first air passage 612 which extends between the air inlet 112 and the heat exchanger unit 108, and the outlet air flow moves along a seconds direction in a second air passage 614 which extends between the heat exchanger unit 108 and the air outlet 114, wherein the second direction is parallel and opposite to the first direction, i.e. the air flow turns an angle of 180 degrees when going from the air inlet 112 to the air outlet 114. In a first experiment 616, the temperature of the exterior surface of the housing 120 of the intraoral scanning device 600 is measured in three different regions of said housing 120: a front end 604 of the housing 120, a central part 606 of the housing 120, and the rear end 608 of the housing 120, and in particular the temperature is measured in a point 604' of the front end 604, in a point 606' of the central part 606, and a point 608' of the rear end 608. The results of the first experiment 616 are illustrated as heat maps, in particular panel 1 shows that the temperature in the point 604' is 49.9 degrees Celsius, panel 2 shows that the temperature in the point 606' is 49.9 degrees Celsius, and panel 3 shows that the temperature in the point 608' is 51.6 degrees Celsius.

The intraoral scanning device 602 is an example of an intraoral scanning device according to the present disclosure. Accordingly, the intraoral scanning device 602 comprises one or more electronic units 104, a heat exchanger unit 108, a fane unit 110, a battery unit 214, an air inlet 112 and an air outlet 114. Differently from the intraoral scanning device 600, the battery unit 214 of the intraoral scanning device 602 is configured to power the fane unit 110, and in particular the fane unit 110 of the intraoral scanning device 602 is mounted, either permanently or detachable, to the battery unit 214. Thus, the battery unit 214 is configured to slide though the heat exchanger unit 108 until at least a part of the fane unit 110 is covered by the heat exchanger unit 108. Furthermore, the air inlet 112 and the air outlet 114 are arranged in vicinity to a rear end 608 of the housing 120 and the air inlet 112 and the air outlet 114 are separated by the heat exchanger unit 108. Referring to the intraoral scanning device 602, the fane unit 110 is configured to suck air through the air inlet 112 along a first direction, let air pass through the heat exchanger unit 108 and blow air out from the air outlet 114 along a second direction, wherein the second direction is the same as the first direction and the first direction and second direction are perpendicular to a longitudinal axis 118 of the intraoral scanning device 602. In a second experiment 618, the temperature of an exterior surface of the housing 120 of the intraoral scanning device 602 is measured in three different regions of said housing 120: a front end 604 of the housing 120, a central part 606 of the housing 120, and the rear end 608 of the housing 120, and in particular the temperature is measured in a point 604' of the front end 604, in a point 606' of the central part 606, and a point 608' of the rear end 608. The results of the second experiment 618 are illustrated as heat maps, in particular panel 1' shows that the temperature in the point 604' is 44.7 degrees Celsius, panel 2' shows that temperature in the point 606' is 43.5 degrees Celsius, and panel 3' shows that temperature in the point 608' is 43.9 degrees Celsius. Thus, the temperature of the exterior surface of the housing 120 of the intraoral scanning device 602 is significantly reduced compared to the temperature of the exterior surface of the housing 120 of the intraoral scanning device 600. It will be appreciated by those skilled in the art that this result is a direct effect of the arrangement of the heat exchanger unit 108, of the fane unit 110, of the air inlet 112 and of the air outlet 114 along the housing 120 of the intraoral scanning device 602, whereby the air flow does not encounter any surface of the housing 120 when going from the air inlet 112 to the heat exchanger unit 108 and when going from the heat exchanger unit 108 to the air outlet 114, thereby preventing the development of air turbulences across the heat exchanger unit 108. Furthermore, while in the intraoral scanning device 600 the inlet air flow moves along a side of the battery unit 214 and it is therefore pre-heated by said battery unit 214 before passing through the heat exchanger unit 108, the inlet air flow does not encounter any heated unit of the intraoral scanning device 602, further contributing to the enhancement of the cooling efficiency of the intraoral scanning device 602. It will be further appreciated that the same results as the ones shown in Fig. 6 for the second experiment 618 may hold for an intraoral scanning device such as the intraoral scanning device as shown as reference number 200 in Fig. 2b (not shown here), wherein the air inlet 112 and the air outlet 114 are arranged on an end-facet of the intraoral scanning device, and wherein the end-facet is arranged at the rear end 608 of the intraoral scanning device, and wherein the heat exchanger unit 108 and the fane unit 106, i.e. the cooling system, are arranged such that a distance of less than 1.5 cm is obtained between the cooling system and the air inlet and the air outlet, respectively. Thus, the intraoral scanning device 602 may be the intraoral scanning device as shown as reference number 100 in Fig. 1, and/or the intraoral scanning device as shown as reference number 200 in Fig. 2, and/or the intraoral scanning device as shown as reference number 300 in Fig. 3.

### Items

1. An intraoral scanning device configured to perform a scanning of a dental object inside an oral cavity, the device comprising:
   - one or more electronic units;
   - a cooling system configured to remove heat from the one or more electronic units, the cooling system comprising a heat exchanger unit and a flow generating unit, wherein at least a part of the flow generating unit is covered by the heat exchanger unit; and
   - an air inlet and an air outlet, wherein the air inlet and air outlet are separated by the heat exchanger unit,
   wherein the flow generating unit is configured to move air between the air inlet and the air outlet and through the heat exchanger unit in a direction mainly perpendicular to a longitudinal axis of the intraoral scanning device.
2. The intraoral scanning device according to item 1, wherein the one or more electronic units comprise:
   - a projector unit that is configured to project light onto the dental object in the oral cavity;
   - an image sensor that is configured to capture reflected light from the dental object in the oral cavity; and
   - one or more processors that are configured to process a plurality of data acquired by the intraoral scanning device during the scanning of the dental object in the oral cavity.
3. The intraoral scanning device according to item 2, wherein the projector unit comprises at least one light source that is configured to generate light of a single wavelength and/or light that is a combination of wavelengths.
4. The intraoral scanning device according to item 3, wherein the at least one light source comprises at least one light emitting diode (LED) unit.
5. The intraoral scanning device according to item 2, wherein the one or more processors comprise a field-programmable gate array (FPGA) and/or an advanced RISC machine (ARM).
6. The intraoral scanning device according to any of the preceding items, comprising a battery unit that is configured to power the flow generating unit via at least one electrical connection or at least one inductive connection.
7. The intraoral scanning device according to any of the preceding items, comprising a battery unit, and wherein the flow generating unit is permanent mounted to the battery unit or the flow generating unit is detachable mounted to the battery unit via a magnetic force or a snap couple.
8. The intraoral scanning device according to any of the preceding items, comprising a battery unit, wherein the heat exchanger is arranged in a rear end of the intraoral scanning device, and wherein the battery unit is configured to slide through the heat exchanger unit until at least a part of the flow generating unit is covered by the heat exchanger unit.
9. The intraoral scanning device according to any of preceding items, wherein the flow generating unit is configured to slide through the heat exchanger unit until at least a part of the flow generating unit is covered by the heat exchanger unit.
10. The intraoral scanning device according to any of the preceding items, comprising a battery unit, wherein the battery unit and the flow generating unit are permanent or detachable mounted to a first housing of the intraoral scanning device, and the battery unit and the flow generating unit are maintained in the first housing when detaching the first housing from a second housing of the intraoral scanning device, and wherein the second housing includes the one or more electronic components.
11. The intraoral scanning device according to item 10, wherein the battery unit or the flow generating unit includes an extractor, and wherein the battery unit and the flow generating unit are arranged within the first housing of the intraoral scanning device, and the extractor is configured to pull out the battery unit and the flow generating unit upon a user pulling the extractor.
12. The intraoral scanning device according to any of the preceding items, comprising a battery unit, wherein the battery unit comprises a Universal Serial Bus (USB) port.
13. The intraoral scanning device according to item 12, wherein the USB port is arranged in an end-facet of the battery, and wherein the end-facet is arranged in vicinity to a rear end of the battery unit.
14. The intraoral scanning device according to item 13, wherein the flow generating unit includes a USB plug configured to be connected to the USB port comprised in the battery unit, and wherein at least one electrical connection between the flow generating unit and the battery unit is obtained by the connection between the USB plug and the USB port.
15. The intraoral scanning device according to item 12, wherein the USB port is arranged in a front-facet of a front end of the battery unit, and wherein the front end is arranged opposite to the rear end of the battery unit.
16. The intraoral scanning device according to item 15, wherein at least one of the one or more electronic units comprises a USB plug configured to be connected to the USB port comprised in the battery unit, and wherein at least one electrical connection between the battery unit and the one or more electronic units is obtained by the connection between the USB plug and the USB port.
17. The intraoral scanning device according to any of the preceding items, wherein the heat exchanger unit comprises two sides, and the heat exchanger unit is arranged such that a first side faces a tip end of the intraoral scanning device, and a second side faces a rear end of the intraoral scanning device, and wherein the heat exchanger unit is coupled to the one or more electronic units through at least one heat transferring unit, and the at least one heat transferring unit is detachable or permanent mounted to the first side of the heat exchanger unit.
18. The intraoral scanning device according to item 17, wherein the second side of the heat exchanger unit is permanent or detachable mounted to a first housing of the intraoral scanning device that is detachable mounted to a second housing of the intraoral scanning device, and wherein the second housing comprises the one or more electronic units.
19. The intraoral scanning device according to any of items17-18, wherein the at least one heat transferring unit includes one or more heat pipes, and the one or more electronic components include a first electronic component and a second electronic component, wherein the one or more heat pipes include a first heat pipe and a second heat pipe, and wherein the first heat pipe is coupled to the first electronic component and the second heat pipe is coupled to the second electronic component.
20. The intraoral scanning device according to any of claims 17-19, comprising a heat dissipation unit that is thermally connected to the one or more electronic units and to the at least one heat transferring unit, wherein the at least one heat transferring unit is permanent mounted to the heat dissipation unit.
21. An intraoral scanning device configured to perform a scanning of a dental object inside an oral cavity, the device comprising:
   - one or more electronic units;
   - a cooling system configured to remove heat from the one or more electronic units, the cooling system comprising a heat exchanger unit and a flow generating unit, wherein at least a part of the flow generating unit is covered by the heat exchanger unit; and
   - an air inlet and an air outlet, and
   wherein the flow generating unit is configured to move air between the air inlet, the air outlet and through the heat exchanger unit, and wherein the cooling system is arranged in vicinity to a rear end of the intraoral scanning device.
22. The intraoral scanning device according to item 21, wherein the cooling system is arranged such that a distance of less than 1.5 cm is obtained between the cooling system and the air inlet and the air outlet, respectively.
23. The intraoral scanning device according to any of items 21-22, wherein the air inlet and the air outlet are arranged in an end-facet of the intraoral scanning device, and wherein the end-facet is arranged at the rear end of the intraoral scanning device.
24. The intraoral scanning device according to any of items 21-23, wherein the air inlet and the air outlet are separated by the cooling system.
25. An intraoral scanning device configured to perform a scanning of a dental object inside an oral cavity, the device comprises a housing, wherein the housing includes:
   - one or more electronic units;
   - a cooling system configured to remove heat from the one or more electronic units, the cooling system comprising a heat exchanger unit and a flow generating unit, wherein at least a part of the flow generating unit is covered by the heat exchanger unit; and
   - one or more processors that is configured to control the fane unit such that a temperature of an exterior surface of the housing is between 40 and 50 degrees Celsius; and
   wherein the intraoral scanning device may include a tip housing attached to the housing, wherein the tip housing includes at least a window.
26. An intraoral scanning device configured to perform a scanning of a dental object inside an oral cavity, the device comprising:
   - one or more electronic units;
   - a cooling system configured to remove heat from the one or more electronic units, the cooling system comprising a heat exchanger unit and a flow generating unit, wherein at least a part of the flow generating unit is covered by the heat exchanger unit; and
   - an air inlet and an air outlet, wherein the air inlet and the air outlet are separated by the heat exchanger, and the air inlet and air outlet are arranged distantly to a tip end of the intraoral scanning device, the tip end comprising a scanning window,
   wherein the flow generating unit is configured to move air between the air inlet and the air outlet and through the heat exchanger unit in a direction mainly perpendicular to a longitudinal axis of the intraoral scanning device.

## Claims

1. An intraoral scanning device (100) configured to perform a scanning of a dental object (102) inside an oral cavity, the device comprising:
- one or more electronic units (104);
- a cooling system (106) configured to remove heat from the one or more electronic units (104), the cooling system (106) comprising a heat exchanger unit (108) and a flow generating unit (110), wherein at least a part of the flow generating (110) unit is covered by the heat exchanger unit (108); and
- an air inlet (112) and an air outlet (114), wherein the air inlet (112) and air outlet (114) are separated by the heat exchanger unit (108),
wherein the flow generating unit (110) is configured to move air between the air inlet (112) and the air outlet (114) and through the heat exchanger unit (108) in a direction (116) mainly perpendicular to a longitudinal axis (118) of the intraoral scanning device (100).

2. The intraoral scanning device according to claim 1, comprising a battery unit that is configured to power the flow generating unit via at least one electrical connection or at least one inductive connection.

3. The intraoral scanning device according to any of the preceding claims, comprising a battery unit, wherein the flow generating unit is permanent mounted to the battery unit or the flow generating unit is detachable mounted to the battery unit via a magnetic force or a snap couple.

4. The intraoral scanning device according to any of the preceding claims, comprising a battery unit, wherein the battery unit is configured to slide through the heat exchanger unit until at least one part of the flow generating unit is covered by the heat exchanger unit.

5. The intraoral scanning device according to any of the preceding claims, wherein the flow generating unit is configured to slide through the heat exchanger unit until at least a part of the flow generating unit is covered by the heat exchanger unit.

6. The intraoral scanning device according to any of the preceding claims, comprising a battery unit, wherein the battery unit and the flow generating unit are permanent or detachable mounted to a first housing of the intraoral scanning device, and the battery unit and the flow generating unit are maintained in the first housing when detaching the first housing from a second housing of the intraoral scanning device, and wherein the second housing includes the one or more electronic components.

7. The intraoral scanning device according to any of the preceding claims, wherein the heat exchanger unit comprises two sides, and the heat exchanger unit is arranged such that a first side faces a tip end of the intraoral scanning device, and a second side faces a rear end of the intraoral scanning device, and wherein the heat exchanger unit is coupled to the one or more electronic units through at least one heat transferring unit, and the at least one heat transferring unit is detachable or permanent mounted to the first side of the heat exchanger unit.

8. The intraoral scanning device according to claim 7, wherein the second side of the heat exchanger unit is mounted to a first housing of the intraoral scanning device that is detachable mounted to a second housing of the intraoral scanning device, and wherein the second housing comprises the one or more electronic units of the device.

9. The intraoral scanning device according to any of claims 7-8, wherein the at least one heat transferring unit includes one or more heat pipes, and the one or more electronic components include a first electronic component and a second electronic component, wherein the one or more heat pipes include a first heat pipe and a second heat pipe, and wherein the first heat pipe is coupled to the first electronic component and the second heat pipe is coupled to the second electronic component.

10. The intraoral scanning device according to any of claims 7-9, comprising a heat dissipation unit that is thermally connected to the one or more electronic units and to the at least one heat transferring unit, and the at least one heat transferring unit is permanent mounted to the heat dissipation unit.

11. The intraoral scanning device according to any of the preceding claims, comprising a battery unit, wherein the battery unit comprises a Universal Serial Bus (USB) port.

12. The intraoral scanning device according to claim 11, wherein the USB port is arranged in an end-facet of the battery, and wherein the end-facet is arranged in vicinity to a rear end of the battery unit.

13. The intraoral scanning device according to claim 12, wherein the flow generating unit includes a USB plug configured to be connected to the USB port comprised in the battery unit, and wherein at least one electrical connection between the flow generating unit and the battery unit is obtained by the connection between the USB plug and the USB port.

14. The intraoral scanning device according to claim 13, wherein the USB port is arranged in a front-facet of a front end of the battery unit, and wherein the front end is arranged opposite to the rear end of the battery unit.

15. The intraoral scanning device according to claim 14, wherein at least one of the one or more electronic units comprises a USB plug configured to be connected to the USB port comprised in the battery unit, and wherein at least one electrical connection between the battery unit and the one or more electronic units is obtained by the connection between the USB plug and the USB port.
